# EUROPEAN PATENT SPECIFICATION

(11) **EP 1 290 158 B1**
(45) Date of publication and mention of the grant of the patent: **06.12.2006**
(21) Application number: 01946058.3
(22) Date of filing: 01.06.2001
(51) Int. Cl.: C12N 15/10

(54) **PROCESSING OF PLASMID-CONTAINING FLUIDS**
BEHANDLUNG VON PLASMID-HALTIGEN LÖSUNGEN
TRAITEMENT DE FLUIDES CONTENANT DES PLASMIDES

(30) Priority: 02.06.2000 US 208561 P
(43) Date of publication of application: 12.03.2003
(73) Proprietor: PALL CORPORATION, East Hills, NY 11548-1209 (US)
(72) Inventor: NOCHUMSON, Samuel, Gulf Breeze, FL 32561 (US); YANG, Yujing, Newton, MA 02460 (US); KINSEY, Joe, L., Jr., Irvington, AL 36544-0934 (US)
(74) Representative: Hoeger, Stellrecht & Partner Patentanwälte
(86) International application number: PCT/US2001/017953
(87) International publication number: WO 2001/094573

(56) References cited:
- EP-A- 0 853 123
- WO-A-00/50161
- WO-A-00/69549
- WO-A-01/79486
- WO-A-97/08306
- WO-A-99/63076

## Description

### TECHNICAL FIELD

This invention relates to processing of plasmid-containing fluids in general, and in particular to purifying or separating plasmids from a plasmid and endotoxin-containing fluid and obtaining a purified plasmid.

### BACKGROUND OF THE INVENTION

Processing of plasmid-containing fluids is of great interest in recent years as highly purified plasmids are becoming increasingly important in, for example, molecular biology and medicine. Highly purified plasmids are useful in a variety of applications in molecular biology, e.g., in sequencing, cloning, and hybridization (e.g., by PCR), and in medicine, e.g., gene therapy and gene immunization. The purity of the plasmid is critical in these applications, particularly in medicine. As the plasmid may be administered to a patient, the plasmid should be of a pharmaceutical grade. The plasmid should meet drug quality standards of safety. Thus, for example, the plasmid should be free of substances, such as endotoxins, that could elicit a toxic response.

Plasmids, which are extrachromosomal DNAs, are generally isolated from plasmid-containing fluids such as bacterial cell lysates. The cell lysates contain a variety of contaminants, e.g., cell debris, protein, RNA, chromosomal DNA, and endotoxins. Methods have been proposed for processing plasmid-containing fluids such as bacterial cell lysates, e.g., for the isolation and purification of plasmids, although many of these methods have drawbacks. In a common method for processing a crude bacterial cell lysate, the crude lysate is centrifuged to remove cell debris. The lysate is then extracted with an organic solvent such as phenol or chloroform to remove the protein contaminant. The RNA content of the lysate is reduced by treatment with enzymes such as RNase.

EP 0 853 123 A1 discloses a process and a device for purification and/or concentration of nucleic acids in a solution. Said process involves the step of cross-flow filtration where the nucleic acids are retained by a membrane and compounds of a lower molecular weight pass through the membrane. Alternatively, the compounds of a lower molecular weight could be adsorbed on the membrane. As a result of this process step purified and/or concentrated nucleic acid solutions are obtained. In a similar way endotoxins may be separated from a nucleic acid preparation.

A buoyant density-based separation method also has been proposed to process plasmid-containing fluids. This method involves mixing a crude sample or preparation containing a plasmid with an intercalating dye, e.g., ethidium bromide, and then overlayering the sample on top of a cesium chloride (CsCl) solution of higher buoyant density. The resulting over-lay is centrifuged at high speeds to form a gradient of CsCl of increasing buoyant density. The dye/plasmid complex separates as a discrete band, the complex is separated, and the plasmid is recovered from the complex.

Processes involving the use of porous beads, ion-exchange resins, and gel filtration media also have been suggested for processing of plasmid-containing fluids.

Many of the foregoing methods have one or more drawbacks. For example, the processed plasmids still include contaminants such as endotoxins. Some of the methods involve multiple steps and are labor intensive or time consuming. As a result, the plasmids, especially the valuable supercoiled form of the plasmid, tend to degrade during the processing. Certain methods require the use of harmful or harsh chemicals or solvents. For example, ethidium bromide is a known mutagen. In some methods, the media employed for the separation are of limited binding affinity and/or capacity for the macromolecule of interest. Some of the methods cannot be scaled-up readily. In certain methods, the materials employed in the process cannot be reused.

Certain other methods produce plasmids that are contaminated with enzymes, as these methods involve the use of enzymes to degrade and remove the RNA contaminant. The residual enzymes increase the risk of contaminating the plasmid with undesirable materials such as RNase. As the enzymes are sometimes obtained from an extraneous source such as an animal, the residual enzymes can cause inter-species contamination if the plasmid is administered to a patient. In addition, as enzymes can digest the plasmid, the quality of the plasmid can deteriorate with time if contaminating enzymes such as DNases are present. Enzymes also can be expensive and drive up the cost of plasmid purification.

Thus, there exists a need for a method (as well as a system and kit) for processing plasmid-containing fluids to obtain plasmids that are free or substantially free of endotoxins. There further exists a need for a method for processing plasmid-containing fluids which does not involve or require the use of harmful or harsh chemicals. There further exists a need for a method for processing plasmid-containing fluids which does not require the use of enzymes.

The present invention provides for ameliorating at least some of the disadvantages of the prior art methods. These and other advantages of the present invention will be apparent from the description set forth below.

### SUMMARY OF THE INVENTION

The present invention provides a method for processing a plasmid-containing fluids. In an embodiment, the present invention provides a method for processing a plasmid and endotoxin-containing fluid comprising contacting the fluid with a first membrane that binds at least a portion of the plasmid and a second membrane that binds at least a portion of the endotoxin, and obtaining a plasmid-enriched and endotoxin-depleted fluid, e.g., a fluid containing purified plasmid.

In another embodiment, the present invention provides a method for processing a plasmid and endotoxin-containing fluid comprising contacting the fluid with at least three membranes, two of which bind at least a portion of the plasmid and one of which binds at least a portion of the endotoxin, and obtaining a plasmid-enriched and endotoxin-depleted fluid.

In a preferred embodiment, the membranes that bind the plasmid and endotoxin are charged membranes. The charged membranes comprise charge-providing groups. Examples of charge-providing groups include ion-exchange groups.

The present invention further provides a system as well as a kit for processing plasmid-containing fluids. The present invention also provides a method for processing a mixture of supercoiled and open circular forms of a plasmid comprising contacting the mixture with a positively charged membrane.

The present invention provides one or more of the following advantages. The method, system, or kit offers highly purified plasmids which are free or substantially free of endotoxins. The plasmids obtained are free of enzyme contamination. The plasmids obtained are free or substantially free of proteins, chromosomal DNA, and/or RNA. The plasmids have a high percentage of the valuable supercoiled form.

The method of the present invention does not require the use of harsh or harmful chemicals. The method does not require the use of enzymes. The method is scaleable. The method can produce small quantities as well as large quantities of highly purified plasmids. The membranes employed in the method are reusable. The method is more rapid, less time-consuming, and less labor-intensive relative to many methods known heretofore. The method of the present invention processes the plasmid-containing fluid with little or no degradation of the plasmids, particularly the supercoiled form of the plasmid.

### BRIEF DESCRIPTION OF THE DRAWINGS

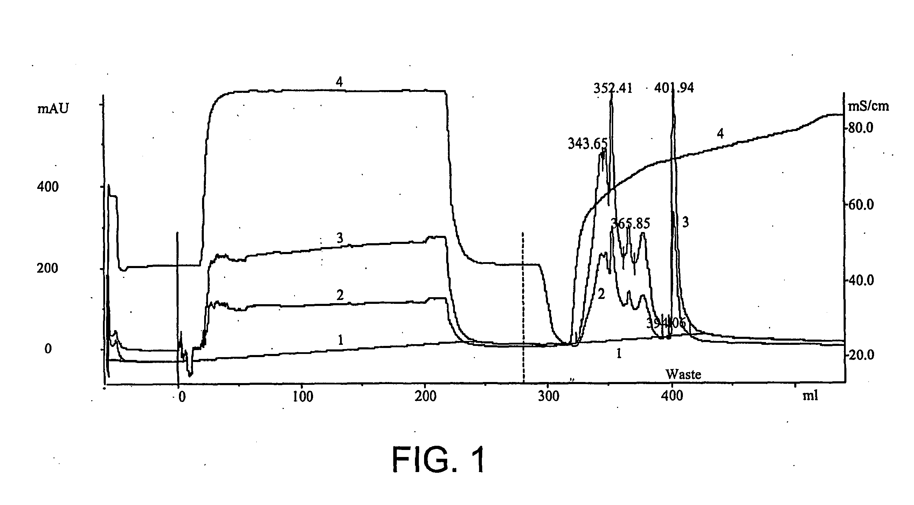

Fig. 1 depicts the composition of the eluate as a function of retention volume from a plasmid binding membrane that is contacted with a bacterial lysate. The x-axis depicts the retention volume in mL. In curves 1-3, the y-axis at the left depicts the absorbance (in milli absorbance units or "mAU") of the eluate at 260 nm (curve 2) and 280 nm (curve 3) and of the base line at 260 nm (curve 1). In curve 4, the y-axis at the right depicts the conductivity (in milli Siemens/cm or "mS/cm") of the eluate. The peak at 401.94 mL corresponds to the plasmid.

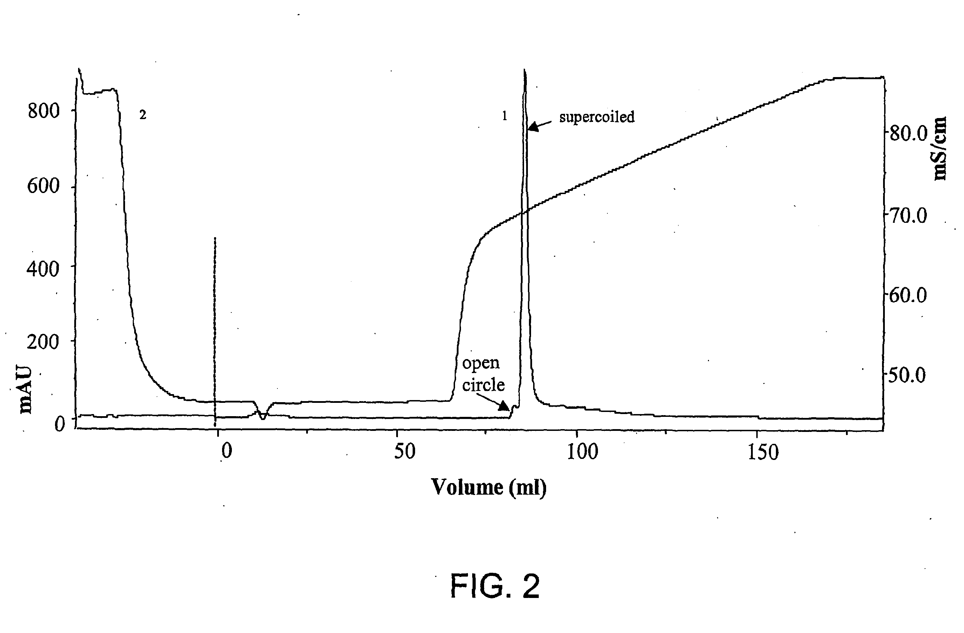

Fig. 2 depicts the composition of the eluate as a function of retention volume from a first positively charged membrane that is contacted with a sample containing supercoiled and open circular forms of a β-galactosidase reporter plasmid. The x-axis depicts the retention volume in mL. The y-axis depicts the absorbance (in mAU) for curve 1, and the conductivity (in mS/cm) for curve 2. The membrane allows separation of the open circular form of the plasmid (small peak) from the supercoiled form of the plasmid (large peak).

### SPECIFIC DESCRIPTION OF THE INVENTION

The present invention provides a method for processing plasmid-containing fluids. The method is rapid and obtains high quality purified plasmids. The plasmids do not degrade, and the obtained plasmids are free or substantially free of endotoxins.

In an embodiment, the present invention provides a method for processing a plasmid and endotoxin-containing fluid comprising contacting the fluid with a first membrane that binds at least a portion of the plasmid and a second membrane that binds at least a portion of the endotoxin, and obtaining a plasmid-enriched and endotoxin-depleted fluid.

In another embodiment, the present invention provides a method for processing a plasmid and endotoxin-containing fluid comprising contacting the fluid with a first membrane and a second membrane which bind at least a portion of the plasmid and a third membrane that binds at least a portion of the endotoxin, and obtaining a plasmid-enriched and endotoxin-depleted fluid. In a particular embodiment, the present invention provides a method for processing a fluid containing a plasmid, a protein, and an endotoxin comprising (a) contacting the fluid with a first membrane to bind at least a portion of the plasmid and the endotoxin; (b) contacting the first membrane from (a) with an eluant to obtain a plasmid and endotoxin-containing eluate; (c) contacting the eluate from (b) with a second membrane that binds at least a portion of the endotoxin; and (d) obtaining a plasmid-enriched and endotoxin-depleted fluid.

The present invention, in yet another embodiment, provides a method for processing a fluid containing a plasmid, a protein, and an endotoxin comprising (a) contacting the fluid with a first membrane to bind at least a portion of the plasmid and the endotoxin; (b) contacting the first membrane from (a) with an eluant to obtain a first eluate containing plasmid and endotoxin, optionally (b') contacting the first eluate with a second membrane to bind at least a portion of the plasmid and the endotoxin and (b") contacting the second membrane in (b') with an eluant to obtain a second eluate containing plasmid and endotoxin; (c) contacting the first or the second eluate with a third membrane to bind at least a portion of the endotoxin; and (d) obtaining a plasmid-enriched and endotoxin-depleted fluid.

The present invention provides, in still another embodiment, a method for processing a first fluid containing a plasmid, an endotoxin, and at least one or more of the contaminants selected from the group consisting of chromosomal DNA, RNA, and protein, the method comprising (a) treating the first fluid with a first membrane to obtain a second fluid which is enriched in the plasmid relative to the first fluid, (b) treating the second fluid with a second membrane to obtain a third fluid that contains plasmid and endotoxin, (c) treating the third fluid with a third membrane to bind at least a portion of the endotoxin, and (d) obtaining a plasmid-enriched and endotoxin-depleted fluid.

In a preferred embodiment, the membranes employed in the methods of the present invention are charged, e.g., contain ion-exchange groups. Accordingly, the present invention provides a method for processing a plasmid-containing fluid comprising contacting a plasmid-containing fluid with a first positively charged membrane and a second charged membrane, and obtaining a purified plasmid. Preferably, the second charged membrane also is positively charged.

In another embodiment, the present invention provides a method for processing a plasmid-containing fluid comprising contacting a plasmid-containing fluid with a first positively charged membrane, a second positively charged membrane, and a third charged membrane, and obtaining a purified plasmid. Preferably, the third charged membrane also is positively charged.

In a particular embodiment, the present invention provides a method for processing a fluid comprising a plasmid, a protein, and an endotoxin, the method comprising (a) contacting the fluid with a first positively charged membrane to bind the plasmid and the endotoxin; (b) contacting the first positively charged membrane from
(a) with an eluant to obtain an eluate containing the plasmid and the endotoxin; (c) contacting the eluate from (b) with a second charged membrane to bind the endotoxin; and (d) obtaining a purified plasmid.

In another embodiment, the present invention provides a method for processing a fluid containing a plasmid, a protein, and an endotoxin comprising (a) contacting the fluid with a first positively charged membrane to bind the plasmid and the endotoxin; (b) contacting the first positively charged membrane from (a) with an eluant to obtain an eluate containing the plasmid and the endotoxin, optionally (b') contacting the eluate obtained in (b) with a second positively charged membrane to bind the plasmid and the endotoxin and (b") contacting the second positively charged membrane in (b') with an eluant to obtain an eluate containing plasmid and the endotoxin; (c) contacting the eluate from (b) or (b") with a third charged membrane to bind the endotoxin; and (d) obtaining a purified plasmid.

In yet another embodiment, the present invention provides a method for processing a first fluid containing a plasmid, an endotoxin, and at least one or more of the contaminants selected from the group consisting of chromosomal DNA, RNA, and protein, the method comprising (a) treating the first fluid with a first positively charged membrane to obtain a second fluid which is enriched in the plasmid and the endotoxin and depleted in one or more of the contaminants, relative to the first fluid, (b) treating the second fluid with a second positively charged membrane to obtain a third fluid containing the plasmid and endotoxin, (c) treating the third fluid with a third charged membrane to bind the endotoxin, and (d) obtaining a purified plasmid.

The plasmid-containing fluid to be processed in accordance with the present invention can include any suitable plasmid. For example, the fluid can be prepared, derived, or obtained from any suitable source such as a prokaryote or an eukaryote, e.g., bacteria or yeast. A preferred plasmid source is *E. coli.* Examples of plasmids include pGEM, pCAT, pUC19, and pBR322. Other examples include F, R1, Col, Col E1, R6, Ent, Cam, and T1. The plasmids can be open circular, linear, nicked, relaxed, or preferably supercoiled. The plasmids can be high copy number, low copy number, or runaway plasmids. They can contain a range of genetic elements that include selectable genes, polylinkers, origins of replication, promoters, enhancers, leader sequences, polyadenylation sites, and/or termination sequences.

The plasmid-containing fluid, e.g., plasmid and endotoxin-containing fluid, can be prepared from bacterial cells, for example, as follows. The bacterial cells are lysed with an alkali solution, e.g., 0.2 M NaOH containing 1% SDS, to obtain a lysate containing the plasmid, chromosomal DNA, RNA, and protein. A major portion of the protein and the chromosomal DNA can be precipitated by adding a salt solution, e.g., acidified potassium acetate such as 3-4 M potassium acetate at pH 5.5. The precipitates can be removed by filtering through a filter such as a cloth or paper filter, and optionally through a microporous membrane such as a 0.2 µm polyethersulfone membrane, e.g., a SUPOR^{™} membrane (Pall Corp., East Hills, NY). In an embodiment, the lysate can be clarified in a filter train comprising a 20 µm filter, a 0.7 µm filter, and a 0.2 to 0.8 µm filter.

In accordance with an embodiment of the invention, the clarified lysate filtrate is contacted with a first membrane, e.g., a first positively charged membrane. The filtrate can be optionally diluted, e.g., with water, prior to contacting with the first membrane. The first membrane binds plasmids, and, in embodiments, the endotoxins. Preferably, the first membrane binds contaminants such as protein, chromosomal DNA, and/or RNA weakly relative to the plasmid, or more preferably, the membrane does not bind the contaminants.

Typically, any bound protein, chromosomal DNA, or RNA can be removed from the first membrane by washing with a buffer having a low ion (or salt) concentration such that the bound plasmid is not removed from the membrane but the contaminants are. For example, a salt solution such as a NaCl solution, of from about 0.5 M to about 0.6 M, and preferably about 0.6 M, in a suitable buffer, e.g., Tris-HCl, can be used to selectively remove the contaminants. The word "elute" in this application refers to processes known in the art including washing, removing, desorbing, and/or extracting.

Subsequent to the removal of the contaminants, the bound plasmid can be eluted with a salt solution, e.g., a NaCl solution, of a greater ion concentration such as, e.g., from about 0.7 to about 1.0 M, and preferably about 0.8 M, in a suitable buffer. An example of a suitable buffer is a Tris buffer such as Tris-HCl at pH 8.0. The buffer can be at a pH of from about 7 to about 9, and preferably at about 9.0. The buffer can have an ion concentration of from about 1 mM to about 50 mM. The buffer can have a conductivity of, e.g., about 70 mS/cm.

Fig. 1 illustrates the composition of the eluate from a first positively charged membrane is contacted with the filtered bacterial (*E. coli*) lysate. The membrane, configured in a 10 mL capacity module, is contacted with the lysate. The membrane is eluted with 0.5 M NaCl in Tris buffer up to a retention volume of approximately 300 mL. Subsequently, a gradient of NaCl, 0.5 M to 1.0 M, in the Tris buffer is employed for the elution. The peak at 401.94 mL corresponds to the plasmid, pGEM. The peaks at the lower retention volumes, 343.65, 352.41, 365.85, and 394.06 mL, correspond to the contaminants including proteins, RNA, and chromosomal DNA. Contaminants eluting at retention volumes of up to about 220 mL are believed to include oligonucleotides.

Optionally, the first membrane (which binds the plasmid) is contacted with a nonionic surfactant. The membrane can be contacted with the nonionic surfactant prior to, or simultaneously with, contacting the plasmid-containing fluid. Alternatively, the membrane can be contacted with the nonionic surfactant by including it in the plasmid-containing fluid. Any suitable nonionic surfactant, e.g., ethoxylated alkyl phenyl ethers, can be used. A particular example of ethoxylated alkyl phenyl ether is polyoxyethylene (10) isooctylphenyl ether, available as TRITON X-100™. For example, the plasmid-containing fluid is diluted with a nonionic surfactant solution, e.g., a 5% TRITON X-100 solution, to a final concentration of about 1% of the nonionic surfactant, prior to loading the plasmid-containing fluid on the membrane. The use of the nonionic surfactant increases the purity of the plasmid. It is believed that the nonionic surfactant prevents or reduces binding of endotoxin on the membrane that binds plasmid.

As a further option, the eluate obtained from the first membrane can be processed, e.g., placed in contact, with a second membrane (which binds plasmids), particularly when the contaminants are excessive, and the purification is repeated. For example, the fraction eluting at or about 401.94 mL (Fig. 1) can be collected and contacted with the second membrane.

In a preferred embodiment of the method, the plasmid-containing fluid to be processed is contacted with the first membrane such that a substantial amount, e.g., 50% or more, of the contaminants are rapidly removed during this aspect of the process. The eluate obtained from the first membrane contains plasmids, endotoxins, and a reduced quantity of the contaminants such as protein, chromosomal DNA, and/or RNA. An optional second membrane is employed to further purify the eluate from the first membrane. Residual contaminants such as protein, chromosomal DNA, and RNA are removed during this optional processing with the second membrane as these contaminants do not bind to the membrane strongly or can be washed off readily. The bound plasmid and endotoxin are eluted from the second membrane with a suitable eluant.

The resulting eluate is enriched in plasmid, and is depleted in protein as well as other contaminants such as chromosomal DNA, RNA, and endotoxins. The plasmid-rich eluate is contacted with a second membrane (which binds endotoxins) in accordance with a method of the present invention. If an optional second membrane is employed to bind the plasmid, the membrane employed to bind the endotoxin should be referred to as a third membrane.

The second membrane which binds the endotoxin (or the third membrane as the case may be) is preferably charged. The second membrane can be positively charged or negatively charged. A positively charged membrane is preferred. The second charged membrane binds endotoxins with a greater affinity and/or capacity than plasmids. The second charged membrane loaded with plasmid, endotoxin, and any other contaminant is washed with a salt solution such as a NaCl solution having a concentration of from about 0.5 M to about 0.6 M, and preferably about 0.6 M to remove the proteins, RNA, and/or chromosomal DNA. This is followed by a gradient elution, wherein the salt concentration is gradually increased, e.g., from about 0.5 M to about 0.8 M. The plasmid elutes during the gradient elution. The plasmid obtained by this process is free or substantially free of endotoxins, e.g., one or more lipopolysaccharides.

The plasmid can be recovered from the eluate by methods known to those skilled in the art, for example, by ultrafiltration/diafiltration, which may be operated, for example, in a tangential flow filtration mode. Preferably, diafiltration is employed to remove the salts present in the plasmid obtained above. Any suitable diafiltration membrane, e.g., an ultrafiltration membrane such as one having a MWCO of from about 30,000 to about 300,000, and preferably from about 70,000 to about 100,000, can be used. Any suitable diafiltration solution, e.g., a 10 mM Tris-HCl at pH 8.0; 1 mM EDTA, can be employed. The resulting plasmid solution can be concentrated to obtain a plasmid concentrate.

The plasmid solution can be concentrated by methods known to those skilled in the art, for example, by tangential flow filtration, e.g., over a nanofiltration or reverse osmosis membrane. The plasmid can be sterilized, e.g., sterile-filtered through a microporous membrane, such as a membrane having a pore rating of from about 0.1 to about 0.2 µm, and preferably about 0.2 µm. The plasmid thus isolated is of high purity (and can be produced with high yield) and is suitable for many critical applications including gene therapy.

The method of the present invention, in one embodiment, is particularly suitable for processing supercoiled plasmid-containing fluids; and in another embodiment, is suitable for processing relaxed plasmid-containing fluids.

The first membrane can be any suitable membrane that has binding capacity for plasmids. Examples of such membranes are disclosed in International Publication No. WO 00/50161, published 31 August 2000. In a preferred embodiment, the first membrane is positively charged, and in a further preferred embodiment, the first positively charged membrane is hydrophilic. In one embodiment, the first positively charged hydrophilic membrane comprises a porous substrate and a crosslinked coating having cationic groups. Preferably, the crosslinked coating comprises a crosslinked polyamine, e.g., a polyalkyleneimine such as polyethyleneimine (PEI).

The cationic group of the first positively charged membrane can be an ammonium, sulfonium, phosphonium, or other group, preferably an ammonium group. A preferred ammonium group is a quaternary ammonium group such as a tetraalkylammonium group. The cationic groups are preferably present as pendant groups. It is believed that the cationic groups, when present as pendant groups, rather than as part of the backbone, provide enhanced binding capacity and/or selectivity.

The cationic group can be linked directly, i.e., without a spacer, to the backbone of the crosslinked coating polymer or through a spacer group. Preferably the linking is through a spacer group. Examples of spacer groups include one or more moieties selected from the group consisting of hydroxy, hydroxyalkyl, amino, aminoalkyl, amido, alkylamido, ester, and alkoxyalkyl. Further examples of spacer groups include one or more moieties selected from the group consisting of hydroxyalkyl, alkylamino, hydroxyalkylaminoalkyl, hydroxyalkylaminoalkyl hydroxyalkyl, alkylaminoalkyl, and alkylamido. A preferred spacer group is hydroxyalkyl. It is believed that the spacer groups provide spatial charge separation and an increased opportunity for the fixed charges to interact with the materials being treated, particularly, the plasmid. The spacer group provides enhanced binding capacity and/or selectivity.

The spacer group can of any suitable length, for example, the spacer group can be a group having from 1 to about 10 atoms, e.g., carbon atoms. Thus, the spacer group can be from 1 to about 10 carbon atoms long, preferably from 2 to about 6 carbon atoms long, and more preferably about 3 carbon atoms long.

The cationic group can be provided by any suitable method. For example, the polyamine can be contacted with a glycidyl compound having a cationic group so that the epoxy ring opens at the primary or secondary amino groups of a polyamine such as a polyalkyleneimine. Further, a solution of a polyamine such as PEI can be combined or reacted with, e.g., glycidyl trimethylammonium chloride, and a polyamine having trimethylammonium chloride pendant groups linked through hydroxyalkyl groups can be obtained.

In an embodiment, the cationic group is linked to PEI through a reaction with a glycidyl compound such as a polyglycidyl compound having a cationic group. An example of a polyglycidyl compound is a polyalkyleneglycol polyglycidylether such as ethyleneglycol diglycidylether or butyleneglycol diglycidylether.

In a preferred embodiment, the first positively charged membrane comprises a hydrophilic porous polyethersulfone substrate and a crosslinked coating comprising the reaction product of a PEI having pendant quaternary ammonium groups and a polyalkyleneglycol polyglycidylether.

One embodiment of the first positively charged membrane can be prepared as follows. The membrane can be prepared by providing a coating composition on the porous substrate, preferably a hydrophilic porous substrate, and curing the coated substrate. The coating composition can be prepared, e.g., by dissolving the polyamine in a suitable solvent. Preferred solvents include water, low boiling alcohols such as methanol, ethanol, and propanol, and combinations thereof. The solvent can be present in an amount of from about 40% to about 99%, and preferably in an amount of from about 90% to about 99% by weight of the coating composition. The polyamine can be present in an amount of from about 1% to about 5%, and preferably in an amount of from about 1% to about 2.5% by weight of the coating composition.

The hydrophilic porous substrate can be made of any suitable material; preferably, the substrate comprises a polymer. Examples of suitable polymers include polyaromatics, polysulfones, polyamides, polyimides, polyolefins, polystyrenes, polycarbonates, cellulosic polymers such as cellulose acetates and cellulose nitrates, fluoropolymers, and PEEK. Aromatic polysulfones are preferred. Examples of aromatic polysulfones include polyethersulfone, bisphenol A polysulfone, and polyphenylsulfone. Polyethersulfone is particularly preferred. The hydrophilic porous substrate can have any suitable pore size, for example, a pore size of from about 0.01 or 0.03 µm to about 10 µm, preferably from about 0.1 µm to about 5 µm, and more preferably from about 0.2 µm to about 5 µm. The hydrophilic porous substrate can be asymmetric or symmetric.

The hydrophilic porous substrate can be prepared by methods known to those of ordinary skill in the art. For example, it can be prepared by a phase inversion process. Thus, a solution containing the polymer, a solvent, a pore former, a wetting agent, and optionally a small quantity of a non-solvent is prepared by combining and mixing the ingredients, preferably at an elevated temperature. The resulting solution is filtered to remove any impurities. The filtered solution is cast or extruded in the form of a sheet or hollow fiber. The resulting sheet or fiber is allowed to set or gel as a phase inverted membrane. The membrane thus set is leached to remove the solvent and other soluble ingredients.

The porous substrate can be coated with the coating composition by methods known to those of ordinary skill in the art, for example, by dip coating, spray coating, meniscus coating, and the like. Dip coating, for example, can be carried out as follows. The substrate is immersed in the composition for a given period of time sufficient to insure coating of the pore walls. The immersion time can be from about 1 second to about 5.0 minutes, preferably from about 1 second to about 1.0 minutes, and more preferably from about 0.1 minute to about 0.3 minute. Following the immersion, the excess coating composition is removed by draining the substrate under gravity or by the use of a squeegee or air knife. The resulting coated substrate is cured to effect the curing or crosslinking of the coating composition. Thus, for example, the membrane can be cured at a temperature of below 130°C, e.g., from about 50°C to about 130°C, and preferably at a temperature of from about 70°C to about 130°C, for a suitable period of time, which can range from about 5 minutes to about 60 minutes, and preferably from about 10 minutes to about 30 minutes. The resulting membrane can be washed to leach out any extractable in the membrane. Illustratively, the membrane can be leached in hot water, e.g., in deionized water held above 73°C. The resulting membrane is dried in air or in an oven to remove the water. The first positively charged membrane thus prepared is capable of binding plasmids. In embodiments, the first positively charged membrane has a plasmid binding capacity of from about 15 to about 25 mg/mL of membrane.

In accordance with an embodiment of the present invention, the second membrane, which selectively binds endotoxins, is charged, and can be positively charged or negatively charged. A positively charged membrane is preferred. Examples of such membranes are disclosed in International Publication No. WO 00/69549, published 23 November 2000. The membrane is preferably a hydrophilic membrane, and more preferably a hydrophilic positively charged microporous membrane.

The second membrane, in an embodiment, includes a porous hydrophobic substrate and a coating comprising a charge-providing agent. The charge-providing agent can be a polymer, e.g., a positively charged polymer containing quaternary ammonium groups. An example of such a polymer is a polyamine containing quaternary ammonium groups. It is further preferred that the polyamine is crosslinked, e.g., through the ring opening reaction of an epoxy group.

Examples of preferred positively charged polymers include PEI, more preferably PEI modified to contain quaternary ammonium groups. Illustratively, a modified PEI can be prepared by reacting PEI with epichlorohydrin such that some or all of the tertiary amino groups of PEI are converted to quaternary ammonium groups. Such epichlorohydrin modified PEIs can also be obtained commercially. For example, LUPASOL^{™} SC-86X is an epichlorohydrin modified PEI available from BASF Corp. in Mount Olive, NJ. Preferably, the positively charged polymer further includes a quaternized polyamine, e.g., polydialkylamime such as polydimethylamine. An example of a quaternized polyamine is poly(dimethylamine-*co*-epichlorohydrin) and is available as Catalog No. 652 from Scientific Polymer Products, Inc., Ontario, NY.

The hydrophobic substrate can comprise any suitable material; preferably, the substrate comprises a hydrophobic polymer. Examples of hydrophobic polymers include polysulfones, polyolefins, polystyrenes, polyaromatics, cellulosics, polyesters, polyamides such as aromatic polyamides and aliphatic polyamides having long alkyl segments, e.g., C₈-C₁₆ segments, polyimides, polytetrafluoroethylene, polycarbonates, and PEEK. Aromatic polysulfones are preferred. Examples of aromatic polysulfones include polyethersulfone, bisphenol A polysulfone, and polyphenylsulfone. Polyethersulfone is particularly preferred. The hydrophobic porous substrate can be asymmetric or symmetric.

The porous hydrophobic substrate can have any suitable pore size, for example, a pore size of about 10 µm or less, e.g., in the range of from about 0.1 µm to about 10 µm, preferably from about 0.1 µm to about 5 µm, and more preferably from about 0.2 µm to about 1 µm.

The porous hydrophobic substrate can be prepared by methods known to those of ordinary skill in the art. For example, it can be prepared by a phase inversion process. Thus, a casting solution containing the hydrophobic polymer, a solvent, a pore former, and optionally a small quantity of a non-solvent is prepared by combining and mixing the ingredients, preferably at an elevated temperature. The resulting solution is filtered to remove any insolubles or impurities. The casting solution is cast or extruded in the form of a sheet or hollow fiber. The resulting sheet or fiber is allowed to set or gel as a phase inverted membrane. The membrane is leached to remove the solvent and other soluble ingredients.

An embodiment of the second membrane (that selectively binds endotoxins) can be prepared as follows. The porous hydrophobic substrate is contacted with a coating composition comprising a charge providing agent or a precursor thereof. The contacting is carried out such that the charge-providing agent or precursor(s) thereof preferably coat the pore walls of the hydrophobic substrate. Thus, for example, the charge-providing agent or its precursor(s) can be dissolved in a suitable solvent that is compatible with the hydrophobic substrate to provide a solution that is subsequently placed in contact with the substrate.

Preferred solvents include water, low boiling alcohols such as methanol, ethanol, and isopropanol, and combinations thereof. Thus, for example, a mixture of water and ethanol is preferred. The solvent or the mixture of solvents is present in an amount of from about 80% to about 99% by weight, and preferably in an amount of from about 88% to about 97% by weight, of the coating composition.

To prepare a positively charged membrane, the polyamine or the mixture of polyamine precursors is typically present in an amount of from about 0.5% to about 20% by weight, and preferably in an amount of from about 1% to about 9% by weight of the coating composition. In addition, the coating composition may contain a pH adjusting agent, e.g., to provide a pH level of from about 9.5 to about 11.5, and preferably from about 10.5 to about 11.0. The pH can be adjusted by the use of a base, e.g., an alkali such as potassium hydroxide.

The porous hydrophobic substrate can be coated with the coating composition by methods known to those of ordinary skill in the art, for example, by dip coating, spray coating, meniscus coating, and the like. Dip coating, for example, can be carried out as follows. The substrate is immersed in the composition for a suitable period of time. For example, the immersion time can be from about 1 second to about 15 minutes, preferably from about 2 seconds to about 15 seconds, and more preferably from about 3 seconds to about 5 seconds.

Following the immersion, the excess coating composition is allowed to drain or is removed, e.g., by the use of a squeegee or air knife. The resulting coated substrate is heated to remove the solvent, and, in certain embodiments, to allow the precursors to cure into a charge-providing polymeric agent. Thus, for example, a water/ethanol solution of the precursors, e.g., epichlorohydrin modified PEI and quaternized poly(dimethylamine-*co*-epichlorohydrin), and a base, for example, potassium hydroxide, is prepared.

A hydrophobic substrate, for example, a porous polyethersulfone sheet, is immersed in the coating composition for about 3 seconds. The excess coating composition is drained or removed, and the substrate is then allowed to cure, e.g., in a convection oven, at a temperature of from about 90°C to about 150°C, and preferably from about 130°C to about 140°C, for a suitable period of time. Thus, for example, the substrate can be cured at 135°C for a period of from about 15 minutes to about 30 minutes. The resulting membrane can be washed or leached to remove any extractable residues in the membrane. Illustratively, the membrane can be leached in boiling deionized water. The resulting membrane is dried in air or in an oven to remove the water.

As discussed, the second membrane binds endotoxins. The ability of the second membrane to bind endotoxins can be demonstrated by any suitable method. Illustratively, the following method is provided. A sample solution containing a plasmid, e.g., pGEM, at a concentration of 23 µg/mL and endotoxin at a concentration of 1.2 x 10³ EU/mL in a 0.75M NaCl-50 mM Tris buffer at pH 8 can be placed in contact with a 25 mm positively charged membrane disc (estimated filtration area = 3.7 cm²). The flow rate of the sample solution can be kept at 1 mL/min (linear flow rate = 0.27 cm/min). One mL fractions of the eluate can be collected, and the endotoxin concentrations determined, e.g., by the standard limulus amebocyte lysate (LAL) assay after 1:10 dilution of the eluate fractions with pyrogen-free water. The detection limit of the LAL assay is 0.5 EU/mL.

Endotoxin is undetectable in the first several, e.g., the first 10, fractions. The concentration of endotoxin is less than 2 EU/mL in the following 10 fractions, less than 4 EU/mL in further 10 fractions, and less than 10 EU/mL in subsequent 10 fractions.

In a preferred embodiment, the second (or third) membrane has an endotoxin binding capacity of at least about 100,000 EU/cm², e.g., from about 120,000 EU/cm² to about 200,000 EU/cm² or greater, and preferably greater than about 130,000 EU/cm², in water as well as in 0.9% saline. In a further preferred embodiment, the membrane has an endotoxin binding capacity of at least about 50,000 EU/cm², e.g., from about 50,000 EU/cm² to about 150,000 EU/cm² or greater, and preferably greater than about 100,000 EU/cm², in a 0.15 M NaCl in 10 mM Tris buffer at pH 8.

The second membrane (that binds endotoxin) is suitable for reducing endotoxin concentration from samples containing plasmids and endotoxins. Embodiments of the membrane can reduce endotoxins present in nucleic acid (e.g., plasmid DNA) samples from over 1000 EU/mL of a fluid to less than 10 EU/mL (>2 logs). The endotoxin concentration can be reduced from over 52,000 EU/mg of plasmid DNA to less than 500 EU/mg plasmid DNA.

In biological samples containing contaminants such as proteins, chromosomal DNA, and others, certain embodiments of the second membrane yield a 20-fold reduction in endotoxin concentration per mg of plasmid. It is contemplated that greater reductions may be achieved by an appropriate choice of process or membrane parameters, e.g., by increasing the membrane area, contacting the first membrane with a nonionic surfactant, and/or diluting the bacterial lysate.

The method of the present invention is suitable for purifying plasmids in a wide range of quantities. For example, plasmid can be purified in nanograms to kilograms quantities. In certain embodiments, plasmids can be obtained or processed in about 1 gram to about 20 gram quantities. The plasmid yields are high, e.g., in certain embodiments, the yield is higher than about 70%, and preferably greater than about 99% by weight.

The purified plasmid has a high content of the supercoiled form. For example, the supercoiled form is greater than about 90%, and preferably greater than about 95% by weight. The relaxed or open circular form is less than about 10%, and preferably less than about 5% by weight.

The method of the present invention produces purified plasmids having very low contents of contaminants. For example, endotoxin is present in an amount of less than about 50 EU/mg, and in some embodiments, in an amount of less than about 10, 2, or 0.4 EU/mg, of plasmid. The chromosomal DNA content is less than about 1%, and preferably less than about 0.5% by weight. The RNA content is less than about 1%, and preferably less than about 0.2% by weight. The protein content is less than about 1%, and preferably less than about 0.1% by weight.

The membranes employed in embodiments of the present invention are specially advantageous because the high surface area is accessible to bind the plasmid or the endotoxin. The porous structure allows relatively unrestricted or free access to the binding sites of the membranes. The pendant ion-exchange groups as well as the hydrophilic nature of the porous substrate of the first positively charged membrane facilitate or enhance the selective binding of plasmids. The ion-exchange groups as well as the hydrophobic nature of the porous substrate of the second charged membrane facilitate or enhance the binding of endotoxins.

The membranes can be configured in any suitable form, e.g., to provide a device. For example, the device can include a plurality of membranes, e.g., to provide a multilayered filter element, or stacked to provide a membrane module, such as a membrane module for use in chromatography. In an embodiment, the device can include two or more types of membranes, for example, a membrane that binds a plasmid and a membrane that binds an endotoxin; a first positively charged membrane and a second charged membrane; or a first positively charged membrane, a second positively charged membrane, and a third charged membrane.

The membranes can be configured in small volume or large volume devices. For example, the first positively charged membrane is preferably configured as a large volume device such as a membrane cartridge, having a volume capacity of from about 1 L or less to about 10 L or more. Examples of suitable cartridge configuration include those disclosed in UK Patent Application GB 2 275 626 A, such as a generally cylindrical medium wherein the membrane is in a pleated form. Multilayered structures involving overlapping layers of membranes can be provided.

Illustratively, the device can include a filter element comprising the first or the second membrane in a substantially planar or pleated form. In an embodiment, the filter element can have a hollow generally cylindrical form. If desired, the device can further include upstream and/or downstream support or drainage layers. The device can include a plurality of membrane layers. For embodiments of the membrane which are in the form of a tube or fiber, bundles of tubes or fibers can be converted into modules by potting their ends, e.g., by the use of an adhesive. Membrane cartridges can be constructed by including a housing and endcaps to provide fluid seal as well as at least one inlet and at least one outlet.

The second membrane is preferably configured as a small volume device such as a module of volume capacity from about 1 mL or more to about 100 mL or more. Examples of suitable configurations are disclosed in International Publication Nos. WO 00/50888 and WO 00/50144, both published 31 August 2000. For example, the module can include a hollow housing member, a plurality of membranes stacked in the hollow housing member, and a sealant disposed between the stacked membranes and the hollow housing member and sealing an outer periphery of the stacked membranes.

The second membrane or the third membrane (e.g., membranes that have greater affinity for endotoxins than for plasmids) can be configured in any suitable form, preferably a small volume device such as the module described above.

The membranes employed in the methods of the present invention can also be configured for use with microtiter plates, e.g., as high throughput microtiter plates. In an embodiment, the membranes can be configured for use as spin columns.

By the use of the membranes in accordance with the present invention greater amounts of plasmids can be purified relative to many methods employing conventional resins or beads as adsorbents. The flow rate of the fluids passed through the membranes in accordance with the invention can be relatively high, and the pressure drop, relatively low. Binding of plasmid on the first positively charged membrane takes place almost instantly. The second charged membrane binds endotoxins almost instantly. As the method can be carried out in a relatively short time, the plasmid does not degrade significantly during the processing. For example, the supercoiled form of the plasmid does not get nicked.

The plasmids obtained by embodiments of the present invention are of high purity and can be used for a number of applications including in restriction enzyme digestion, cloning, PCR, ligation, and sequencing as well as in gene therapy.

The present invention further provides a system for processing a plasmid-containing fluid comprising at least two membranes as described above, e.g., a first membrane and a second membrane, such as a first positively charged membrane and a second charged membrane. In an embodiment, the system comprises a first positively charged membrane that includes a porous substrate and a crosslinked coating having pendant cationic groups, and a second charged membrane. In another embodiment, the system for processing a fluid containing a plasmid comprising a first positively charged membrane, and a second charged microporous membrane that is hydrophilic and includes a porous hydrophobic substrate and a coating comprising a charge-providing agent. In a further embodiment, the system includes a first positively charged membrane that includes a porous substrate and a crosslinked coating having pendant cationic groups, and a second charged microporous membrane that is hydrophilic and includes a porous hydrophobic substrate and a coating comprising a charge-providing agent.

The system can include additionally, an arrangement for providing the plasmid-containing fluid to the first membrane; an arrangement for contacting the plasmid-containing eluate with the second membrane; and arrangements for eluting the first membrane and the second membrane.

The present invention further provides a system including membrane devices, e.g., membrane based chromatography devices comprising a membrane that binds a plasmid and a membrane that binds an endotoxin, such as a first positively charged membrane and a second charged membrane. The devices can be in any suitable form. For example, the devices comprise a housing including at least one inlet and at least one outlet defining a fluid flow path between the inlet and the outlet, and a membrane disposed across the fluid flow path (crossflow) or tangentially to the fluid flow path (tangential flow). The fluid to be treated can be passed, for example, tangentially to the membrane surface (e.g., allowing a portion of the fluid to pass from the first surface to the second surface to a first outlet, and allowing another portion to pass across the first surface to a second outlet), or passed perpendicular to the membrane surface.

The present invention further provides a kit for processing a plasmid-containing fluid. The kit comprises a membrane that binds a plasmid and a membrane that binds an endotoxin, e.g., a first positively charged membrane and a second charged membrane, and one or more buffer and salt solutions. The membranes can be provided as small, easy-to-use chromatography devices.

The present invention also provides a method for processing a fluid containing a mixture of different forms of plasmid, e.g., a mixture of supercoiled and open circular forms of the plasmid. The method comprises contacting the fluid for processing with a first positively charged membrane. The bound plasmids can be eluted, i.e., removed or washed off the membrane by the use of a suitable eluant, e.g., NaCl solution, in a suitable buffer, e.g., Tris. Various forms of the plasmid can be separated from one another, e.g., the supercoiled from the relaxed or open circular form, by adjusting the conditions of binding and elution, e.g., the type and concentration of the salt employed in the elution. The condition or environment for preferably binding the supercoiled form over the relaxed open circular form includes providing a salt/buffer solution such as about 0.62 M NaCl in 10 mM Tris-HCl buffer at pH 8.0. Alternatively, or in addition, the electrical conductivity of the salt/buffer solution is from about 60 to about 64 mS/cm.

Fig. 2 depicts the composition of the eluate as a function of retention volume from a first positively charged membrane that is contacted with a sample containing supercoiled and open circular forms of a β-galactosidase reporter plasmid. The membrane comprises a polyethersulfone support and a crosslinked coating of PEI having quaternary ammonium groups, and the coating is crosslinked through the use of ethyleneglycol diglycidylether.

The sample contains about 95% by weight supercoiled form and about 5% by weight open circular form. As can be seen from Fig. 2, the membrane separates the open circular form of the plasmid (small peak) from the supercoiled form of the plasmid (large peak). The resolution is excellent. The method is scaleable from a small scale to a large scale, e.g., to a preparative scale.

The following example further illustrates the present invention, but of course, should not be construed as in any way limiting its scope.

### EXAMPLE

This Example illustrates a method of processing a plasmid-containing fluid in accordance with an embodiment of the invention.

20 grams of *E. coli* cells containing a pGEM plasmid are suspended in 150 mL of 50 mM Tris buffer at pH 8.0 containing 10 mM EDTA per gram of cells. The cells are lysed by adding an equal volume of 0.2 M NaOH with 1% SDS for a period of about 3-5 minutes with gentle mixing. A major fraction of protein, chromosomal DNA, and RNA contaminants are precipitated by the addition of 300 mL of 4 M potassium acetate solution. The resulting crude lysate is filtered through a DACRON^{™} fabric and through a 0.2 µm SUPOR CAP™ (Pall Corp., East Hills, NY) filter cartridge.

The filtrate obtained above is diluted with water to a conductivity of approximately 90 mS/cm. The diluted filtrate is loaded onto a cartridge containing a first positively charged membrane comprising a polyethersulfone support and a crosslinked coating of PEI having quaternary ammonium groups, and the coating is crosslinked through the use of ethyleneglycol diglycidylether. The first positively charged membrane has a plasmid binding capacity of 20 mg/mL.

Membrane bound contaminants are washed off with a 0.60 M NaCl solution in 10 mM Tris buffer at pH 8.0. The bound plasmid is eluted with 1.0 M NaCl in 10 mM Tris buffer at pH 8.0, and collected as a plasmid pool for further processing.

The plasmid pool obtained above is optionally diluted with water to a conductivity of 50 mS/cm and loaded onto a 10 mL capacity chromatography module containing a first positively charged membrane as above. The module is washed with a 0.5 M NaCl solution to remove proteins, RNA, and chromosomal DNA, followed by a gradient elution. The supercoiled plasmid elutes during the gradient elution at an eluant conductivity of from about 67 to about 70 mS/cm.

The plasmid pool obtained from the gradient eluate is diluted with an equal volume of water and passed through a third charged membrane comprising a porous hydrophobic polyethersulfone substrate and a crosslinked coating comprising epichlorohydrin modified PEI and quaternized poly(dimethylamine-*co*-epichlorohydrin). This membrane binds the endotoxin. The filtrate contains highly purified plasmid. The plasmid is free or substantially free of RNA, and no RNase is employed in the process. The plasmid is also free or substantially free of proteins.

The plasmid containing filtrate is subjected to diafiltration through a membrane having a MWCO of about 100,000. The resulting plasmid solution is concentrated by tangential flow filtration. The concentrate is sterile filtered. The resulting plasmid is suitable for use in many pharmaceutical applications.

## Claims

1. A method for processing a fluid containing a plasmid and an endotoxin comprising (a) contacting the fluid with a first membrane to bind at least a portion of the plasmid and the endotoxin; (b) contacting the first membrane from (a) with an eluant to obtain a plasmid and endotoxin-containing eluate; (c) contacting the eluate from (b) with a second membrane that binds at least a portion of the endotoxin; and (d) obtaining a plasmid-enriched and endotoxin-depleted fluid.

2. A method for processing a fluid containing a plasmid and an endotoxin comprising (a) contacting the fluid with a first membrane to bind at least a portion of the plasmid and the endotoxin; (b) contacting the first membrane from (a) with an eluant to obtain a first eluate containing plasmid and endotoxin, (b') contacting the first eluate with a second membrane to bind at least a portion of the plasmid and the endotoxin and (b'') contacting the second membrane from (b') with an eluant to obtain a second eluate containing plasmid and endotoxin; (c) contacting the second eluate with a third membrane to bind at least a portion of the endotoxin; and (d) obtaining a plasmid-enriched and endotoxin-depleted fluid.

3. The method of claim 1 or 2, wherein the fluid for processing includes a plasmid, an endotoxin, and at least one or more of the contaminants selected from the group consisting of chromosomal DNA, RNA, and protein, the method comprising treating the fluid with a first membrane to bind at least a portion of the plasmid, the endotoxin and at least one of the contaminants.

4. The method of claim 3, wherein the fluid for processing includes at least one of a chromosomal deoxyribonucleic acid, a ribonucleic acid and a protein.

5. The method of any of claims 1-4, which includes recovering a purified plasmid.

6. The method of claim 5, wherein the purified plasmid comprises a supercoiled plasmid.

7. The method of any of claims 1-6, wherein the fluid for processing comprises a bacterial lysate.

8. The method of any of claims 1-7, wherein the second membrane is a positively charged membrane.

9. The method of any of claims 1 to 8, wherein the first membrane is a positively charged membrane.

10. The method of claim 8 or 9, wherein the positively charged membrane comprises a porous substrate and a crosslinked coating having cationic groups.

11. The method of claim 10, wherein the crosslinked coating comprises a crosslinked polyamine.

12. The method of claim 11, wherein the crosslinked polyamine includes a polyalkyleneimine.

13. The method of any of claims 10 to 12, wherein the cationic group includes a quaternary ammonium group.

14. The method of any of claims 10 to 13, wherein the cationic group is linked to a spacer group.

15. The method of any of claims 12 to 14, wherein the cationic group is linked to the polyalkyleneimine through reaction with a glycidyl compound.

16. The method of any of claims 12 to 15, wherein the coating is crosslinked through the use of a polyglycidyl compound.

17. The method of claim 2, wherein the second membrane comprises a hydrophilic porous polyethersulfone substrate and a crosslinked coating comprising the reaction product of a polyethyleneimine having quaternary ammonium groups and a polyalkyleneglycol polyglycidylether.

18. The method of any one of claims 1 to 7 and 17, wherein the first membrane comprises a hydrophilic porous polyethersulfone substrate and a cross-linked coating comprising a reaction product of a polyethyleneimine having quaternary ammonium groups and a polyalkyleneglykol polyglycidylether.

19. The method of any of claims 1 and 3 to 7, wherein the second membrane a hydrophilic charged membrane.

20. The method of any one of claims 2 to 7, wherein the third membrane comprises a hydrophilic charged membrane.

21. The method of claim 19 or 20, wherein the hydrophilic charged membrane includes a porous hydrophobic substrate and a coating comprising a charge-providing agent

22. The method of claim 21, wherein the charge-providing agent comprises a positive charge-providing agent.

23. The method of claim 22, wherein the positive charge-providing agent comprises a positively charged polymer.

24. The method of claim 23, wherein the positively charged polymer comprises quaternary ammonium groups.

25. The method of claim 23 or 24, wherein the positively charged polymer comprises a polyamine containing quaternary ammonium groups.

26. The method of claim 25, wherein the polyamine is crosslinked.

27. The method of any of claims 10 to 16 and 21 to 26, wherein the porous substrate of the membrane that binds at least a portion of the plasmid or the endotoxin comprises a substrate polymer.

28. The method of claim 27, wherein the substrate polymer is selected from the group consisting of polyaromatics, polysulfones, polyolefins, polystyrenes, polyamides, polyimides, fluoropolymers, polycarbonates, polyesters, cellulose acetates, and cellulose nitrates.

29. The method of any of claims 1-28, which includes contacting the first membrane with a nonionic surfactant.

30. The method of any one of claims 2 to 29, which includes contacting the second membrane with a nonionic surfactant.

31. The method of claim 29 or 30, wherein the nonionic surfactant is an ethoxylated alkyl phenyl ether.

32. The method of claim 31, wherein the ethoxylated alkyl phenyl ether is polyoxyethylene (10) isooctylphenyl ether.

33. The method of any one of claims 1 to 32, wherein the fluid for processing contains supercoiled and open circular forms of a plasmid.

34. The method of claim 33, which includes separating the supercoiled form from the open circular form of the plasmid.

35. The method of any one of claims 1 - 34, wherein the first or the second membrane is a pleated membrane.

36. A system for processing a plasmid and endotoxin-containing fluid comprising a first membrane and a second membrane, wherein the first membrane binds at least a portion of the plasmid and the second membrane binds at least a portion of the endotoxin.

37. The system of claim 36, which includes an additionne membrane which binds at least a portion of the plasmid.

38. The system of claim 36, wherein the first membrane includes a porous substrate and a crosslinked coating having cationic groups.

39. The system of claim 36, wherein the second membrane is hydrophilic and includes a porous hydrophobic substrate and a coating comprising a charge-providing agent.

40. The system of claim 36, wherein the first membrane includes a porous substrate and a crosslinked coating having cationic groups, and the second membrane is hydrophilic and includes a porous hydrophobic substrate and a coating comprising a charge-providing agent.

41. The system of any of claims 36 - 40, including one or more buffers, and/or one or more salt solutions.

42. The system of any one of claims 36 - 41, wherein the first or the second membrane is a pleated membrane.

## Patentansprüche

1. Verfahren zum Behandeln eines Fluids, welches ein Plasmid und ein Endotoxin enthält, umfassend (a) Inkontaktbringen des Fluids mit einer ersten Membran, um mindestens einen Teil des Plasmids und des Endotoxins zu binden; (b) Inkontaktbringen der ersten Membran aus (a) mit einem Eluenten, um ein plasmid- und endotoxinhaltiges Eluat zu erhalten; (c) Inkontaktbringen des Eluats aus (b) mit einer zweiten Membran, die mindestens einen Teil des Endotoxins bindet; und (d) Gewinnen eines plasmidangereicherten und endotoxinverarmten Fluids.

2. Verfahren zum Behandeln eines Fluids, welches ein Plasmid und ein Endotoxin enthält, umfassend (a) Inkontaktbringen des Fluids mit einer ersten Membran, um mindestens einen Teil des Plasmids und des Endotoxins zu binden; (b) Inkontaktbringen der ersten Membran aus (a) mit einem Eluenten, um ein erstes plasmid- und endotoxinhaltiges Eluat zu erhalten; (b') Inkontaktbringen des ersten Eluats mit einer zweiten Membran, um mindestens einen Teil des Plasmids und des Endotoxins zu binden; und (b") Inkontaktbringen der zweiten Membran aus (b') mit einem Eluenten, um ein zweites plasmid- und endotoxinhaltiges Eluat zu erhalten; (c) Inkontaktbringen des zweiten Eluats mit einer dritten Membran, um mindestens einen Teil des Endotoxins zu binden; und (d) Gewinnen eines plasmidangereicherten und endotoxinverarmten Fluids.

3. Verfahren nach Anspruch 1 oder 2, wobei das zu behandelnde Fluid aufweist: ein Plasmid, ein Endotoxin und mindestens einen oder mehr der Kontaminanten, die ausgewählt sind aus der aus chromosomaler DNA, RNA und Protein bestehenden Gruppe, wobei das Verfahren umfasst: Behandeln des Fluids mit einer ersten Membran, um mindestens einen Teil des Plasmids, des Endotoxins und mindestens eines der Kontaminanten zu binden.

4. Verfahren nach Anspruch 3, wobei das zu behandelnde Fluid mindestens eine der Komponenten aufweist, welche sind chromosomale Desoxyribonucleinsäure, Ribonucleinsäure und Protein.

5. Verfahren nach einem der Ansprüche 1 bis 4, umfassend das Wiedergewinnen eines gereinigten Plasmids.

6. Verfahren nach Anspruch 5, wobei das gereinigte Plasmid ein "supercoiled" Plasmid umfasst.

7. Verfahren nach einem der Ansprüche 1 bis 6, wobei das zu behandelnde Fluid ein Bakterienlysat umfasst.

8. Verfahren nach einem der Ansprüche 1 bis 7, wobei die zweite Membran eine positiv geladene Membran ist.

9. Verfahren nach einem der Ansprüche 1 bis 8, wobei die erste Membran eine positiv geladene Membran ist.

10. Verfahren nach Anspruch 8 oder 9, wobei die positiv geladene Membran ein poröses Substrat und eine vernetzte Beschichtung mit kationischen Gruppen umfasst.

11. Verfahren nach Anspruch 10, wobei die vernetzte Beschichtung ein vernetztes Polyamin umfasst.

12. Verfahren nach Anspruch 11, wobei das vernetzte Polyamin ein Polyalkylenimin umfasst.

13. Verfahren nach einem der Ansprüche 10 bis 12, wobei die kationische Gruppe eine quaternäre Ammoniumgruppe umfasst.

14. Verfahren nach einem der Ansprüche 10 bis 13, wobei die kationische Gruppe mit einer Spacer-Gruppe verknüpft ist.

15. Verfahren nach einem der Ansprüche 12 bis 14, wobei die kationische Gruppe durch Reaktion mit einer Glycidyl-Verbindung mit dem Polyalkylenimin verknüpft ist.

16. Verfahren nach einem der Ansprüche 12 bis 15, wobei die Beschichtung durch die Verwendung einer Polyglycidyl-Verbindung vernetzt ist.

17. Verfahren nach Anspruch 2, wobei die zweite Membran umfasst: ein hydrophiles poröses Polyethersulfon-Substrat und eine vernetzte Beschichtung umfassend das Reaktionsprodukt von einem Polyethylenimin mit quaternären Ammoniumgruppen und einem Polyalkylenglycolpolyglycidylether.

18. Verfahren nach einem der Ansprüche 1 bis 7 und 17, wobei die erste Membran umfasst: ein hydrophiles poröses Polyethersulfon-Substrat und eine vernetzte Beschichtung umfassend ein Reaktionsprodukt von einem Polyethylenimin mit quaternären Ammoniumgruppen und einem Polyalkylenglycolpolyglycidylether.

19. Verfahren nach einem der Ansprüche 1 und 3 bis 7, wobei die zweite Membran eine hydrophile geladene Membran umfasst.

20. Verfahren nach einem der Ansprüche 2 bis 7, wobei die dritte Membran eine hydrophile geladene Membran umfasst.

21. Verfahren nach Anspruch 19 oder 20, wobei die hydrophile geladene Membran ein poröses hydrophobes Substrat und eine Beschichtung, welche ein Ladung stellendes Agens umfasst, aufweist.

22. Verfahren nach Anspruch 21, wobei das Ladung stellende Agens ein positive Ladung stellendes Agens umfasst.

23. Verfahren nach Anspruch 22, wobei das positive Ladung stellende Agens ein positiv geladenes Polymer umfasst.

24. Verfahren nach Anspruch 23, wobei das positiv geladene Polymer quaternäre Ammoniumgruppen umfasst.

25. Verfahren nach 23 oder 24, wobei das positiv geladene Polymer ein quaternäre Ammoniumgruppen enthaltendes Polyamin umfasst.

26. Verfahren nach Anspruch 25, wobei das Polyamin vernetzt ist.

27. Verfahren nach einem der Ansprüche 10 bis 16 und 21 bis 26, wobei das poröse Substrat der Membran, die mindestens einen Teil des Plasmids oder des Endotoxins bindet, ein Substratpolymer umfasst.

28. Verfahren nach Anspruch 27, wobei das Substratpolymer ausgewählt ist aus der Gruppe, welche aus Polyaromaten, Polysulfonen, Polyolefinen, Polystyrolen, Polyamiden, Polyimiden, Fluorpolymeren, Polycarbonaten, Polyestern, Celluloseacetaten und Cellulosenitraten besteht.

29. Verfahren nach einem der Ansprüche 1 bis 28, umfassend das Inkontaktbringen der ersten Membran mit einem nichtionischen Tensid.

30. Verfahren nach einem der Ansprüche 2 bis 29, umfassend das Inkontaktbringen der zweiten Membran mit einem nichtionischen Tensid.

31. Verfahren nach Anspruch 29 oder 30, wobei das nichtionische Tensid ein ethoxylierter Alkylphenylether ist.

32. Verfahren nach Anspruch 31, wobei der ethoxylierter Alkylphenylether Polyoxyethylen-(10)-isooctylphenylether ist.

33. Verfahren nach einem der Ansprüche 1 bis 32, wobei das zu behandelnde Fluid "supercoiled" und offene zirkuläre Formen eines Plasmids enthält.

34. Verfahren nach Anspruch 33, umfassend das Abtrennen der "supercoiled" Form von der offenen zirkulären Form des Plasmids.

35. Verfahren nach einem der Ansprüche 1 bis 34, wobei die erste oder die zweite Membran eine Faltenmembran ist.

36. System zum Behandeln eines plasmid- und endotoxinhaltigen Fluids, umfassend eine erste Membran und eine zweite Membran, wobei die erste Membran mindestens einen Teil des Plasmids bindet und wobei die zweite Membran mindestens einen Teil des Endotoxins bindet.

37. System nach Anspruch 36, welches eine zusätzliche Membran aufweist, die mindestens einen Teil des Plasmids bindet.

38. System nach Anspruch 36, wobei die erste Membran ein poröses Substrat und eine vernetzte Beschichtung mit kationischen Gruppen aufweist.

39. System nach Anspruch 36, wobei die zweite Membran hydrophil ist und ein poröses hydrophobes Substrat und eine Beschichtung, welche ein Ladung stellendes Agens umfasst, aufweist.

40. System nach Anspruch 36, wobei die erste Membran ein poröses Substrat und eine vernetzte Beschichtung mit kationischen Gruppen aufweist und wobei die zweite Membran hydrophil ist und ein poröses hydrophobes Substrat und eine Beschichtung, welche ein Ladung stellendes Agens umfasst, aufweist.

41. System nach einem der Ansprüche 36 bis 40, umfassend einen oder mehr Puffer und/oder eine oder mehr Salzlösungen.

42. System nach einem der Ansprüche 36 bis 41, wobei die erste oder die zweite Membran eine Faltenmembran ist.

## Revendications

1. Procédé servant à traiter un fluide contenant un plasmide et une endotoxine comprenant (a) de mettre en contact le fluide avec une première membrane pour fixer au moins une partie du plasmide et de l'endotoxine ; (b) de mettre en contact la première membrane provenant de (a) avec un éluant pour obtenir un éluat contenant le plasmide et l'endotoxine ; (c) de mettre en contact l'éluat provenant de (b) avec une deuxième membrane qui fixe au moins une partie de l'endotoxine ; et (d) d'obtenir un fluide enrichi en plasmide et appauvri en endotoxine.

2. Procédé servant à traiter un fluide contenant un plasmide et une endotoxine comprenant (a) de mettre en contact le fluide avec une première membrane pour fixer au moins une partie du plasmide et de l'endotoxine ; (b) de mettre en contact la première membrane provenant de (a) avec un éluant pour obtenir un premier éluat contenant le plasmide et l'endotoxine, (b') de mettre en contact le premier éluat avec une deuxième membrane pour fixer au moins une partie du plasmide et de l'endotoxine et (b") de mettre en contact la deuxième membrane provenant de (b') avec un éluant pour obtenir un deuxième éluat contenant le plasmide et l'endotoxine ; (c) de mettre en contact le deuxième éluat avec une troisième membrane pour fixer au moins une partie de l'endotoxine ; et (d) d'obtenir un fluide enrichi en plasmide et appauvri en endotoxine.

3. Procédé de la revendication 1 ou 2, dans lequel le fluide destiné à être traité comprend un plasmide, une endotoxine et au moins un ou plusieurs des contaminants choisis dans le groupe constitué d'un ADN chromosomique, d'un ARN et d'une protéine, le procédé comprenant de traiter le fluide avec une première membrane pour fixer au moins une partie du plasmide, de l'endotoxine et d'au moins l'un des contaminants.

4. Procédé de la revendication 3, dans lequel le fluide destiné à être traité comprend au moins l'un d'un acide désoxyribonucléique chromosomique, d'un acide ribonucléique et d'une protéine.

5. Procédé de l'une quelconque des revendications 1-4, lequel comprend de récupérer un plasmide purifié.

6. Procédé de la revendication 5, dans lequel le plasmide purifié comprend un plasmide surenroulé.

7. Procédé de l'une quelconque des revendications 1-6, dans lequel le fluide destiné à être traité comprend un lysat bactérien.

8. Procédé de l'une quelconque des revendications 1-7, dans lequel la deuxième membrane est une membrane positivement chargée.

9. Procédé de l'une quelconque des revendications 1 à 8, dans lequel la première membrane est une membrane positivement chargée.

10. Procédé de la revendication 8 ou 9, dans lequel la membrane positivement chargée comprend un substrat poreux et un revêtement réticulé ayant des groupes cationiques.

11. Procédé de la revendication 10, dans lequel le revêtement réticulé comprend une polyamine réticulée.

12. Procédé de la revendication 11, dans lequel la polyamine réticulée comprend une polyalkylèneimine.

13. Procédé de l'une quelconque des revendications 10 à 12, dans lequel le groupe cationique comprend un groupe ammonium quaternaire.

14. Procédé de l'une quelconque des revendications 10 à 13, dans lequel le groupe cationique est lié à un groupe d'intercalation.

15. Procédé de l'une quelconque des revendications 12 à 14, dans lequel le groupe cationique est lié à la polyalkylèneimine grâce à une réaction avec un composé glycidyle.

16. Procédé de l'une quelconque des revendications 12 à 15, dans lequel le revêtement est réticulé grâce à l'utilisation d'un composé polyglycidyle.

17. Procédé de la revendication 2, dans lequel la deuxième membrane comprend un substrat en polyéthersulfone poreux hydrophile et un revêtement réticulé comprenant le produit de la réaction d'une polyéthylèneimine ayant des groupes ammoniums quaternaires et d'un éther de polyalkylèneglycol et de polyglycidyle.

18. Procédé de l'une quelconque des revendications 1 à 7 et 17, dans lequel la première membrane comprend un substrat en polyéthersulfone poreux hydrophile et un revêtement réticulé comprenant un produit de réaction d'une polyéthylèneimine ayant des groupes ammoniums quaternaires et d'un éther de polyalkylèneglycol et de polyglycidyle.

19. Procédé de l'une quelconque des revendications 1 et 3 à 7, dans lequel la deuxième membrane comprend une membrane hydrophile chargée.

20. Procédé de l'une quelconque des revendications 2 à 7, dans lequel la troisième membrane comprend une membrane hydrophile chargée.

21. Procédé de la revendication 19 ou 20, dans lequel la membrane hydrophile chargée comprend un substrat hydrophobe poreux et un revêtement comprenant un agent fournissant des charges.

22. Procédé de la revendication 21, dans lequel l'agent fournissant des charges comprend un agent fournissant des charges positives.

23. Procédé de la revendication 22, dans lequel l'agent fournissant des charges positives comprend un polymère positivement chargé.

24. Procédé de la revendication 23, dans lequel le polymère positivement chargé comprend des groupes ammoniums quaternaires.

25. Procédé de la revendication 23 ou 24, dans lequel le polymère positivement chargé comprend une polyamine contenant des groupes ammoniums quaternaires.

26. Procédé de la revendication 25, dans lequel la polyamine est réticulée.

27. Procédé de l'une quelconque des revendications 10 à 16 et 21 à 26, dans lequel le substrat poreux de la membrane qui fixe au moins une partie du plasmide ou de l'endotoxine comprend un polymère substrat.

28. Procédé de la revendication 27, dans lequel le polymère substrat est choisi dans le groupe constitué de polyaromatiques, de polysulfones, de polyoléfines, de polystyrènes, de polyamides, de polyimides, de polymères fluorés, de polycarbonates, de polyesters, d'acétates de cellulose et de nitrates de cellulose.

29. Procédé de l'une quelconque des revendications 1-28, lequel comprend de mettre en contact la première membrane avec un tensioactif non ionique.

30. Procédé de l'une quelconque des revendications 2 à 29, lequel comprend de mettre en contact la deuxième membrane avec un tensioactif non ionique.

31. Procédé de la revendication 29 ou 30, dans lequel le tensioactif non ionique est un éther d'alkylphényle éthoxylé.

32. Procédé de la revendication 31, dans lequel l'éther d'alkylphényle éthoxylé est l'éther d'isooctylphényle et de polyoxyéthylène (10).

33. Procédé de l'une quelconque des revendications 1 à 32, dans lequel le fluide destiné à être traité contient les formes surenroulée et circulaire ouverte d'un plasmide.

34. Procédé de la revendication 33, lequel comprend de séparer la forme surenroulée de la forme circulaire ouverte du plasmide.

35. Procédé de l'une quelconque des revendications 1-34, dans lequel la première ou la deuxième membrane est une membrane plissée.

36. Système servant à traiter un fluide contenant un plasmide et une endotoxine comprenant une première membrane et une deuxième membrane, dans lequel la première membrane fixe au moins une partie du plasmide et la deuxième membrane fixe au moins une partie de l'endotoxine.

37. Système de la revendication 36, lequel comprend une membrane supplémentaire qui fixe au moins une partie du plasmide.

38. Système de la revendication 36, dans lequel la première membrane comprend un substrat poreux et un revêtement réticulé ayant des groupes cationiques.

39. Système de la revendication 36, dans lequel la deuxième membrane est hydrophile est comprend un substrat hydrophobe poreux et un revêtement comprenant un agent fournissant des charges.

40. Système de la revendication 36, dans lequel la première membrane comprend un substrat poreux et un revêtement réticulé ayant des groupes cationiques et la deuxième membrane est hydrophile et comprend un substrat hydrophobe poreux et un revêtement comprenant un agent fournissant des charges.

41. Système de l'une quelconque des revendications 36-40, comprenant un ou plusieurs tampons et/ou une ou plusieurs solutions de sels.

42. Système de l'une quelconque des revendications 36-41, dans lequel la première ou la deuxième membrane est une membrane plissée.
